# EUROPÄISCHE PATENTSCHRIFT

(11) **EP 1 186 292 B1**
(45) Veröffentlichungstag und Bekanntmachung des Hinweises auf die Patenterteilung: **11.05.2005**
(21) Anmeldenummer: 01119312.5
(22) Anmeldetag: 10.08.2001
(51) Int. Cl.: A61K 9/16, A23L 1/275, A61K 9/20

(54) **Carotinoidtrockenpulver mit einer Multi-Kern Struktur**
Dry carotinoid powder with a mulit-core structure
Pudre sechée des carotinoides avec des noyaux multiples

(30) Priorität: 30.08.2000 DE 10042833
(43) Veröffentlichungstag der Anmeldung: 13.03.2002
(73) Patentinhaber: BASF AKTIENGESELLSCHAFT, 67056 Ludwigshafen (DE)
(72) Erfinder: Auweter, Helmut, Dr., 67117 Limburgerhof (DE); Jensen, Nina Musaeus, 2900 Hellerup (DK); Lüddecke, Erik, Dr., 67112 Mutterstadt (DE); Runge, Frank, Dr., 67159 Friedelsheim (DE)

(56) Entgegenhaltungen:
- EP-A- 0 595 030
- FR-A- 2 524 311
- US-A- 5 780 056

## Beschreibung

Die Erfindung betrifft feste Zubereitungen mindestens zweier, für den Lebensmittel- und Tierfuttermittelbereich oder für pharmazeutische und kosmetische Anwendungen geeigneter Wirkstoffe mit einer Multi-Kern Struktur, insbesondere Carotinoid-haltige Trockenpulver, Verfahren zu deren Herstellung sowie die Verwendung dieser festen Zubereitungen zur Herstellung von Nahrungsergänzungsmitteln sowie als Zusatz zu Lebensmitteln, Tierfuttermitteln, pharmazeutischen und kosmetischen Präparaten.

Bei der Verwendung von festen Zubereitungen, beispielsweise Mischungen von fettlöslichen Vitaminen und/oder von Carotinoiden, deren Zusammensetzungen dem physiologischen Bedarf angepaßt sind und bei denen die Einzelkomponenten teilweise im extremen Über- bzw. Unterschuß vorliegen, werden hohe Anforderungen an die Konfektionierung gestellt. Für den Anwender ist es in diesem Fall besonders wichtig, daß neben der gewünschten Stabilität in allen Partikeln eine homogene Gleichverteilung der Wirkstoffe gewährleistet ist.

Für die Formulierung von Carotinoiden sind in der Patentliteratur eine Reihe von Methoden bekannt.

So beschreiben EP-A-0 065 193 und EP-A-0 937 412 Verfahren zur Überführung von Carotinoiden in feinverteilte, pulverförmige Formen.

EP-A-0 498 824 offenbart ein Mahlverfahren von Carotinoiden in einem Schutzkolloid-haltigen wäßrigen Medium und anschließende Überführung der Dispersion in ein Trockenpulver.

EP-A-0 410 236 betrifft ein Verfahren zur Herstellung von kolloid-dispersen Carotinoidpräparaten durch in Kontaktbringen einer Suspension eines Carotinoids in einem hochsiedenden Öl mit überhitztem Wasserdampf, Emulgieren dieses Gemisches in eine wäßrige Schutzkolloidlösung und anschließende Trocknung.

WO 98/26008 beschreibt ein Verfahren zur Herstellung von stabilen wäßrigen Dispersionen und Trockenpulvern von Xanthophyllen.

In WO 99/48487 werden Zubereitungen von Carotinoid-Mischungen beschrieben, bei denen die Carotinoide aus natürlichen Quellen stammen. Aufgrund des hohen Phospholipidanteils in diesen Zubereitungen, verbunden mit einer hohen Viskosität der öligen Dispersion, sind die anwendungstechnischen Eigenschaften dieser Formulierung nicht immer zufriedenstellend.

Die o.g. Zubereitungen führen im Falle von Carotinoid-Mischungen nicht selten zu Stabilitäts- und Bioverfügbarkeitsproblemen. Ferner kann es bei Mischungen mit extrem unterschiedlichen Mengenanteilen der einzelnen Carotinoide durch Aggregatbildung der Carotinoide untereinander zu unerwünschten inhomogenen Verteilungen der Wirkstoffe in diesen Zubereitungen kommen. Darüber hinaus zeigen Mischungen von Trockenpulvern einzelner Carotinoide während der Lagerung oder beim Transport ebenfalls häufig Entmischungserscheinungen.

Es bestand daher die Aufgabe, feste Wirkstoff-Zubereitungen für Anwendungen im Lebensmittel- und Tierernährungsbereich sowie für pharmazeutische und kosmetische Anwendungen vorzuschlagen, bei denen neben der gewünschten Stabilität, eine homogene Gleichverteilung der Wirkstoffe in allen Partikeln gewährleistet ist.

Diese Aufgabe wurde erfindungsgemäß gelöst durch feste Zubereitungen mindestens zweier, für den Lebensmittel- und Tierfuttermittelbereich oder für pharmazeutische und kosmetische Anwendungen geeigneter Wirkstoffe in Form einer Multi-Kern Struktur, dadurch gekennzeichnet, daß mindestens zwei Kerne einer Multi-Kern Struktur eine unterschiedliche chemische Zusammensetzung aufweisen.

Unter dem Begriff der Multi-Kern Struktur ist im Rahmen der vorliegenden Erfindung eine Partikelspezies (Sekundärpartikel) mit einer mittleren Teilchengröße von 5 bis 3000 µm, bevorzugt 10 bis 2500 µm, besonders bevorzugt 50 bis 2000 µm, ganz besonders bevorzugt 100 bis 1000 µm, zu verstehen, in der eine weitere Partikelspezies (Primärpartikel) - sogenannte Kerne - in einer Matrix eingebettet ist, wobei die Kerne eine mittlere Partikelgröße von bevorzugt 0,01 bis 1,0 µm, besonders bevorzugt 0,03 bis 0,5 µm, ganz besonders bevorzugt 0,05 bis 0,2 µm, aufweisen.

Beispiele solcher Multi-Kern Strukturen finden sich u.a. in US 5,780,056 und in den dort erläuterten Abbildungen sowie in D. Horn und E. Lüddecke: "Preparation and characterization of nano-sized carotenoid hydrosols" in Fine Particle Science and Technology, 761-775 [E. Pelizzetti (Ed.), Kluwer Academic Publishers, Netherlands, 1996] und H. Auweter et al., Angew. Chem. Int. Ed. 1999, 38(5), 2188-91.

Die bislang bekannten Multi-Kern Strukturen sind dadurch gekennzeichnet, daß deren Primärpartikel (siehe oben) in ihrer Zusammensetzung identisch sind, d.h. daß im Falle einer Mischung - beispielsweise von Carotinoiden und/oder Vitaminen - jeder Kern hinsichtlich der Art und Menge der darin enthaltenen Carotinoid/Vitamin-Einzelkomponenten identisch ist.

Die erfindungsgemäßen festen Zubereitungen zeichnen sich u.a. nun dadurch aus, daß sie einerseits durch die Verkapselung der einzelnen Wirkstoffe unerwünschte Wechselwirkungen zwischen den Wirkstoffen innerhalb der Multi-Kern Struktur vermeiden bzw. verringern und andererseits eine flexiblere Gestaltung bei der Herstellung anwenderfreundlicher Rezepturen wirkstoffhaltiger Mischungen ermöglichen.

Als für den Lebensmittel- und Tierernährungsbereich oder für pharmazeutische und kosmetische Anwendungen geeignete Wirkstoffe sind im Rahmen der vorliegenden Erfindung u.a. folgende Verbindungen zu verstehen:

Fettlösliche Vitamine, wie z.B. die K-Vitamine, Vitamin A und Derivate wie Vitamin A-Acetat, Vitamin A-Propionat oder Vitamin A-Palmitat, Vitamin D₂ und Vitamin D₃ sowie Vitamin E und Derivate. Vitamin E steht in diesem Zusammenhang für natürliches oder synthetisches α-, β-, γ- oder δ-Tocopherol, bevorzugt für natürliches oder synthetisches α-Tocopherol sowie für Tocotrienol. Vitamin E-Derivate sind z.B. Tocopheryl-C₁-C₂₀-Acylester wie Tocopherylacetat oder Tocopherylpalmitat.

Wasserlösliche Vitamine, insbesondere Ascorbinsäure und deren Salze wie Natriumascorbat sowie Vitamin C-Derivate wie Natrium-, Calcium- oder Magnesium-ascorbyl-2-monophosphat oder Calciumascorbyl-2-polyphosphat, Calcium-pantothenat, Panthenol, Vitamin B₁ (Thiamin) - als Hydrochlorid, Nitrat oder Pyrophosphat, Vitamin B₂ (Riboflavin) und deren Phosphate, Vitamin B₆ und Salze, Vitamin B₁₂, Biotin, Folsäure und Folsäurederivate wie Tetrahydrofolsäure, 5-Methyltetrahydrofolsäure, 5-Formyltetrahydrofolsäure, Nicotinsäure und Nicotinsäureamid.

Verbindungen mit Vitamin- oder Coenzymcharakter, z.B. Cholinchlorid, Carnitin, γ-Butyrobetain, Liponsäure, Kreatin, Ubichinone, S-Methylmethionin, S-Adenosylmethionin.

Mehrfach ungesättigte Fettsäuren, z.B. Linolsäure, Linolensäure; Arachidonsäure, Eicosapentaensäure, Docosahexaensäure.

Lebensmittelfarbstoffe wie Curcumin, Carmin oder Chlorophyll. Carotinoide, sowohl Carotine als auch Xanthophylle, wie z.B. β-Carotin, Lycopin, Lutein, Astaxanthin, Zeaxanthin, Capsanthin, Capsorubin, Cryptoxanthin, Citranaxanthin, Canthaxanthin, Bixin, β-Apo-4-carotinal, β-Apo-8-carotinal und β-Apo-8-carotinsäureester.

Bevorzugte Ausführungsformen der erfindungsgemäßen festen Zubereitungen sind Carotinoid-haltige Trockenpulver in Form der o.g. Multi-Kern Struktur, die mindestens zwei der o.g. Carotinoide, ausgewählt aus der Gruppe der Carotine und Xanthophylle enthalten.

Besonders bevorzugt sind solche Trockenpulver, die dadurch gekennzeichnet sind, daß mindestens zwei Kerne (Primärteilchen) ein oder mehrere unterschiedliche Carotinoide enthalten. Insbesondere zeichnen sich die Zubereitungen dadurch aus, daß mindestens zwei Kerne nur einen Vertreter aus der Stoffklasse der Carotinoide enthalten.

Die in den Kernen enthaltenen Carotinoide können sowohl natürlichen als auch synthetischen Ursprungs sein. Sie weisen in der Regel eine Reinheit von mindestens 80%, bevorzugt größer 90%, besonders bevorzugt größer 95%, ganz besonders bevorzugt größer 98 % auf, bestimmt durch quantitative HPLC-Analyse.

Bei Carotinoiden aus natürlichen Quellen, z.B. Lutein oder Lycopin ist es möglich, daß diese bis zu 20 % weitere Carotinoide als "Verunreinigungen" enthalten.

Als bevorzugte Carotinoide sind Carotine wie β-Carotin und Lycopin oder Xanthophylle wie Astaxanthin, Lutein, Zeaxanthin und Canthaxanthin zu nennen.

Ganz besonders bevorzugt sind Trockenpulver, enthaltend eine Mischung aus β-Carotin, Lycopin und Lutein.

Ein solches Trockenpulver beinhaltet eine Multi-Kern Struktur aus Sekundärteilchen, in denen mindestens drei Primärteilchen eine unterschiedliche Carotinoid-Zusammensetzung aufweisen, wobei jeweils eine Partikelspezies nur β-Carotin, die zweite Lycopin und die dritte nur Lutein enthält.

Der Gehalt an β-Carotin, Lycopin und Lutein liegt in den erfindungsgemäßen Trockenpulvern im allgemeinen zwischen 0,1 und 50 Gew.-%, bevorzugt zwischen 1 und 35 Gew.-%, besonders bevorzugt zwischen 5 und 25 Gew.-%, ganz besonders bevorzugt zwischen 8 und 20 Gew.-%, bezogen auf die Gesamtmenge der Formulierung.

Im Falle der oben genannten Dreierkombination liegt das Mengenverhältnis der in dem Trockenpulver enthaltenen Carotinoide bei 1 Teil β-Carotin, 0,02 bis 20 Teile Lycopin und 0,02 bis 20 Teile Lutein, bevorzugt bei 1 Teil β-Carotin, 0,1 bis 5 Teile Lycopin und 0,1 bis 5 Teile Lutein, besonders bevorzugt bei 1 Teil β-Carotin, 0,2 bis 2 Teile Lycopin und 0,1 bis 2 Teile Lutein, ganz besonders bevorzugt bei 1 Teil β-Carotin, 0,3 bis 1,2 Teile Lycopin und 0,1 bis 0,8 Teile Lutein.

Die Carotinoid-Formulierungen, insbesondere die o.g. Dreierkombination zeichnen sich weiterhin dadurch aus, daß der Phosphorgehalt in den Formulierungen kleiner 2,0 Gew.-%, vorteilhaft kleiner 1,0 Gew.-%, bevorzugt kleiner 0,5 Gew.-%, besonders bevorzugt kleiner 0,1 Gew.-%, ganz besonders bevorzugt kleiner 0,02 Gew.-%, bezogen auf die Gesamtmenge des Gemisches aus β-Carotin, Lycopin und Lutein ist.

Mit dem geringen Phosphorgehalt ist gleichzeitig auch ein geringer Anteil an Phospholipiden verbunden, wodurch die anwendungstechnischen Eigenschaften der Trockenpulver, wie beispielsweise die Fließfähigkeit in ölhaltigen Dispersionen besonders bei niedrigen Temperaturen verbessert werden.

Die Carotinoid-Formulierungen können in ihren Sekundärpartikeln neben den bereits beschriebenen Carotinoid-haltigen Kernen weitere Primärpartikel enthalten, deren Wirkstoffe nicht aus der Stoffklasse der Carotinoide stammen. Hierbei handelt es sich bevorzugt um Vitamin-haltige Primärpartikel.

Die erfindungsgemäßen Zubereitungen sind ferner dadurch gekennzeichnet, daß die Primärpartikel eine Kern/Schale-Struktur aufweisen, bei der der Wirkstoff-haltige Kern von einem Schutzkolloid umhüllt ist.

Geeignete Schutzkolloide sind sowohl elektrisch geladene Polymere (Polyelektrolyte) als auch neutrale Polymere. Typische Beispiele sind u.a. Gelatine wie Rinder-, Schweine- oder Fischgelatine, Stärke, Dextrin, Pflanzenproteine wie Sojaproteine, die gegebenenfalls hydrolysiert sein können, Pektin, Guar gum, Xanthan, Gummi-Arabikum, Kasein, Kaseinat oder Mischungen davon. Es können aber auch Polyvinylalkohol, Polyvinylpyrrolidon, Methylcellulose, Carboxymethylcellulose, Hydroxypropylcellulose, Blätterschellack und Alginate eingesetzt werden. Bezüglich näherer Einzelheiten wird auf R.A. Morton, Fat Soluble Vitamins, Intern. Encyclopedia of Food and Nutrition, Bd.9, Pergamon Press 1970, S. 128-131, verwiesen.

Bevorzugte Schutzkolloide sind Verbindungen, ausgewählt aus der Gruppe, bestehend aus Gelatine wie Rinder-, Schweine- und Fischgelatine, Pflanzenproteine, Pektin, Kasein, Kaseinat, Gummi Arabicum und Schellack. Besonders bevorzugt eingesetzte Schutzkolloide sind wäßrige Lösungen von Gelatine, Pektin, Kasein, Kaseinat, Gummi Arabicum und/oder Fischgelatine.

Zur Erhöhung der mechanischen Stabilität des Trockenpulvers ist es zweckmäßig, dem Kolloid einen Weichmacher zuzusetzen, wie Zucker oder Zuckeralkohole, z.B. Saccharose, Glucose, Lactose, Invertzucker, Sorbit, Mannit oder Glycerin, aber auch Polymere wie Polyvinylalkohol oder Polyvinylpyrrolidon. Bevorzugt verwendete Weichmacher sind Saccharose, Sorbit und Lactose.

Das Verhältnis von Schutzkolloid und Weichmacher zu Wirkstoff wird im allgemeinen so gewählt, daß man eine feste Zubereitung erhält, die zwischen 0,1 und 50 Gew.-% mindestens zweier Wirkstoffe, 10 bis 50 Gew.-%, bevorzugt 15 bis 35 Gew.-% eines Schutzkolloids und 20 bis 70 Gew.-%, bevorzugt 30 bis 60 Gew.-% eines Weichmachers enthält, wobei sich alle Prozentangaben auf die Trockenmasse der Formulierung beziehen und die Summe der Prozentangaben der Einzelkomponenten 100% ergibt.

Zur Erhöhung der Stabilität der Wirkstoffe gegen oxidativen Abbau kann es vorteilhaft sein, 0 bis 10 Gew.-%, bevorzugt 0,5 bis 7,5 Gew.-%, bezogen auf die Trockenmasse der Formulierung, eines oder mehrerer Stabilisatoren wie α-Tocopherol, t-Butyl-hydroxytoluol, t-Butylhydroxyanisol, Ascorbinsäure oder Ethoxyquine zuzusetzen.

Weiterhin können Emulgatoren beispielsweise Ascorbylpalmitat, Polyglycerin-Fettsäureester, Sorbitan-Fettsäureester, Propylenglycol-Fettsäureester oder Lecithin in einer Konzentration von 0 bis 200 Gew.-%, vorzugsweise 5 bis 150 Gew.-%, besonders bevorzugt 10 bis 80 Gew.-%, bezogen auf die eingesetzten Wirkstoffe verwendet werden.

Unter Umständen kann es auch vorteilhaft sein, zusätzlich ein physiologisch zugelassenes Öl wie beispielsweise Sesamöl, Maiskeimöl, Baumwollsaatöl, Sojabohnenöl oder Erdnußöl sowie Ester mittelkettiger pflanzlicher Fettsäuren in einer Konzentration von 0 bis 500 Gew.%, vorzugsweise 10 bis 300 Gew.%, besonders bevorzugt 20 bis 100 Gew.%, bezogen aufdie Wirkstoffe zu verwenden.

Die in der Multi-Kern Struktur vorliegende Matrix wird in der Regel aus einem physiologisch unbedenklichen polymeren Material gebildet. Bevorzugt setzt sie sich zusammen aus mindestens einem der o.g. Schutzkolloide, gegebenenfalls in Kombination mit den bereits beschriebenen Formulierhilfsstoffen wie Weichmacher, Antioxidantien und/oder Emulgatoren. Die Matrix kann außerdem noch mindestens ein wasserlösliches Vitamin enthalten.

Gegenstand der Erfindung ist auch ein Verfahren zur Herstellung der eingangs beschriebenen festen Zubereitungen durch Trocknung einer wäßrigen Dispersion, enthaltend mindestens zwei, für den Lebensmittel- und Tierfuttermittelbereich oder für pharmazeutische und kosmetische Anwendungen geeignete Wirkstoffe in Form von nanopartikulären Teilchen, dadurch gekennzeichnet, daß mindestens zwei der nanopartikulären Teilchen eine unterschiedliche chemische Zusammensetzung aufweisen. Unter Wirkstoffe sind dabei die bereits eingangs genannten Verbindungen zu verstehen.

Eine bevorzugte Ausführungsform des Verfahrens ist dadurch gekennzeichnet, daß es sich bei den Wirkstoffen um mindestens zwei Carotinoide handelt, wobei besonders bevorzugt mindestens zwei der nanopartikulären Teilchen ein oder mehrere unterschiedliche Carotinoide enthalten.

Eine ganz besonders bevorzugte Verfahrensvariante ist dadurch gekennzeichnet, daß mindestens zwei der nanopartikulären Teilchen nur einen Vertreter aus der Stoffklasse der Carotinoide enthalten.

Aus Stabilitätsgründen ist es hierbei vorteilhaft, wenn die Wirkstoffe in Form von Schutzkolloid-stabilisierten nanopartikulären Teilchen vorliegen, die eine mittlere Partikelgröße von bevorzugt 0,01 bis 1,0 µm, besonders bevorzugt 0,03 bis 0,5 µm, ganz besonders bevorzugt 0,05 bis 0,2 µm, aufweisen.

Die für die Herstellung der erfindungsgemäßen Zubereitungen verwendeten Wirkstoffe, insbesondere die Carotinoide können in Form feinstgemahlener Kristalle oder bevorzugt in Form bereits vorpräparierter Trockenpulver eingesetzt werden. Diese Trocken-. pulver enthalten jeweils nanopartikuläre Teilchen der einzelnen Carotinoide und lassen sich durch Mahlung oder Mikronisierung der einzelnen Wirkstoffe herstellen. Beispiele hierfür finden sich u.a. in EP-A-0 065 193, EP-A-0 937 412 sowie in WO 91/06292.

Durch Redispergierung dieser Ausgangsformulierungen in wäßrigen Lösungen und erneute Überführung der Dispersion in ein Trockenpulver nach an sich bekannten Verfahren wie z.B. der Sprühtrocknung oder der Sprühkühlung, gegebenenfalls unter Zusatz von Puderungsmitteln zur Vermeidung von Agglomeration, lassen sich die neuen erfindungsgemäßen Zubereitungen mit den eingangs beschriebenen Multi-Kern Strukturen erhalten. Einzelheiten zur Sprühtrocknung bzw. Sprühkühlung finden sich u.a. in WO 91/06292

Die erfindungsgemäßen Carotinoid-Formulierungen eignen sich u.a. als Zusatzstoff zur Färbung von Lebensmittelzubereitungen, insbesondere von Getränkezubereitungen, als Mittel für die Herstellung pharmazeutischer und kosmetischer Zubereitungen sowie für die Herstellung von Nahrungsergänzungspräparaten im Humanund Tierbereich.

So lassen sich zum Färben von Getränken beispielsweise die erfindungsgemäßen wasser-dispergerbaren Trockenpulver verwenden, in denen Mischungen von β-Carotin, Lycopin und Lutein in den bereits oben genannten Konzentrationen vorliegen.

Es ist ebenfalls möglich, Trockenpulver, die die erfindungsgemäßen Carotinoidkombinationen enthalten, zur Anreicherung in Milchprodukten wie Joghurt, Milchmixgetränken oder Milchspeiseeis sowie Puddingpulvern, Backmischungen und Süßwaren, beispielsweise Fruchtgummis zu verwenden.

Gegenstand der Erfindung sind auch Nahrungsergänzungsmittel, Tierfuttermittel, Lebensmittel sowie pharmazeutische und kosmetische Zubereitungen, enthaltend die oben beschriebenen Zubereitungen, insbesondere Carotinoid-Formulierungen von Gemischen aus β-Carotin, Lycopin und Lutein.

Unter Nahrungsergänzungspräparate sowie pharmazeutische Zubereitungen, die die erfindungsgemäßen Trockenpulver enthalten, sind u.a. Tabletten, Dragees sowie Hart- und Weichgelatinekapseln zu verstehen. Bevorzugte Nahrungsergänzungspräparate sind Tabletten, in die die Trockenpulver mit eingearbeitet werden sowie Weichgelatinekapseln, in denen die Carotinoid-haltigen Multi-Kern Strukturen als ölhaltige Suspension in den Kapseln vorliegen. Der Gehalt an Carotinoiden in diesen Kapseln liegt im Bereich von 0,5 bis 20 mg β-Carotin, 0,5 bis 20 mg Lycopin und 0,5 bis 20 mg Lutein, bevorzugt im Bereich von 1 bis 15 mg β-Carotin, 1 bis 15 mg Lycopin und 1 bis 10 mg Lutein, besonders bevorzugt im Bereich von 2 bis 10 mg β-Carotin, 2 bis 10 mg Lycopin und 1 bis 5 mg Lutein.

In den folgenden Beispielen wird die Herstellung der erfindungsgemäßen Trockenpulver näher erläutert.

### Beispiel 1

500 g β-Carotin-haltiges Trockenpulver mit einem β-Carotin-Gehalt von 20 Gew.-%, 500 g Lycopin-haltiges Trockenpulver mit einem Lycopin-Gehalt von 10 Gew.-% und 200 g Lutein-haltiges Trockenpulver mit einem Lutein-Gehalt von 10 Gew.-% (alle Trockenpulver hergestellt gemäß EP-B-0 065 193) wurden bei 65°C unter Rühren in 1800 ml Wasser redispergiert. Nachdem die Pulver-Matrix vollständig gelöst war, wurde die Viskosität der Dispersion durch Zugabe von Wasser auf einen Wert von ca. 180 cP (gemessen bei 65°C) eingestellt. Anschließend wurde die Dispersion durch Sprühkühlung und anschließender Trocknung in ein Pulver überführt. Mittel HPLC wurde folgender Gehalt an Carotinoiden im Trockenpulver bestimmt:

| | |
|---|---|
| β-Carotin | 5,3 Gew.-% |
| Lycopin | 3,0 Gew.-% |
| Lutein | 1,1 Gew.-% |
| Gesamtgehalt an Carotinoiden | 9,4 Gew.-% |

### Beispiel 2

Analog Beispiel 1 wurden 575 g β-Carotin-haltiges Trockenpulver mit einem β-Carotin-Gehalt von 20 Gew.-%, 500 g Lycopin-haltiges Trockenpulver mit einem Lycopin-Gehalt von 10 Gew.-% und 200 g Lutein-haltiges Trockenpulver mit einem Lutein-Gehalt von 10 Gew.-% in 1900 ml Wasser redispergiert und anschließend getrocknet. Man erhielt ein Trockenpulver folgender Carotinoid-Zusammensetzung:

| | |
|---|---|
| β-Carotin | 5,7 Gew.-% |
| Lycopin | 2,9 Gew.-% |
| Lutein | 1,1 Gew.-% |
| Gesamtgehalt an Carotinoiden | 9,7 Gew.-% |

### Beispiel 3

Analog Beispiel 1 wurden 700 g β-Carotin-haltiges Trockenpulver mit einem β-Carotin-Gehalt von 20 Gew.-% und 600 g Lutein-haltiges Trockenpulver mit einem Lutein-Gehalt von 10 Gew.-% in 1800 ml Wasser redispergiert und anschließend getrocknet. Man erhielt ein Trockenpulver folgender Carotinoid-Zusammensetzung:

| | |
|---|---|
| β-Carotin | 7,1 Gew.-% |
| Lutein | 3,5 Gew.-% |
| Gesamtgehalt an Carotinoiden | 10,6 Gew.-% |

## Patentansprüche

1. Trockenpulver mindestens zweier Carotinoide in Form einer Multi-Kern Struktur, **dadurch gekennzeichnet, dass** mindestens zwei Kerne einer Multi-Kern Struktur eine unterschiedliche chemische Zusammensetzung aufweisen.

2. Trockenpulver nach Anspruch 1, **dadurch gekennzeichnet, dass** die Multi-Kern Struktur eine Partikelspezies mit einer mittleren Teilchengröße von 5 bis 3000 µm darstellt, in der die Kerne in einer Matrix eingebettet sind.

3. Trockenpulver nach einem der Ansprüche 1 oder 2, bei denen die Kerne eine mittlere Partikelgröße von 0,01 bis 1,0 µm aufweisen.

4. Trockenpulver nach einem der Ansprüche 1 bis 3, **dadurch gekennzeichnet, dass** mindestens zwei Kerne ein oder mehrere unterschiedliche Carotinoide enthalten.

5. Trockenpulver nach einem der Ansprüche 1 bis 4, **dadurch gekennzeichnet, dass** mindestens zwei Kerne nur einen Vertreter aus der Stoffklasse der Carotinoide enthalten.

6. Trockenpulver nach einem der Ansprüche 1 bis 5, **dadurch gekennzeichnet, dass** die Carotinoide eine Auswahl aus der Gruppe der Carotine und Xanthophylle darstellen.

7. Trockenpulver nach einem der Ansprüche 1 bis 6, enthaltend β-Carotin, Lycopin und Lutein.

8. Trockenpulver nach Anspruch 7, enthaltend 1 Gew.-Teil β-Carotin, 0,02 bis 20 Gew.-Teile Lycopin und 0,02 bis 20 Gew.-Teile Lutein.

9. Trockenpulver nach einem der Ansprüche 1 bis 8, mit einem Gehalt an Carotinoiden von 0,1 bis 50 Gew.-%, bezogen auf die Gesamtmenge des Trockenpulvers.

10. Verfahren zur Herstellung von Trockenpulvern, definiert gemäß Anspruch 1, durch Trocknung einer wäßrigen Dispersion, enthaltend mindestens zwei Carotinoide in Form von nanopartikulären Teilchen, **dadurch gekennzeichnet, dass** mindestens zwei der nanopartikulären Teilchen eine unterschiedliche chemische Zusammensetzung aufweisen.

11. Verfahren nach Anspruch 10, **dadurch gekennzeichnet, dass** mindestens zwei der nanopartikulären Teilchen ein oder mehrere unterschiedliche Carotinoide enthalten.

12. Verfahren nach einem der Ansprüche 10 oder 11, **dadurch gekennzeichnet, dass** mindestens zwei der nanopartikulären Teilchen nur einen Vertreter aus der Stoffklasse der Carotinoide enthalten.

13. Verfahren nach einem der Ansprüche 10 bis 12, **dadurch gekennzeichnet, dass** die Carotinoide in Form von Schutzkolloid-stabilisierten nanopartikulären Teilchen vorliegen.

14. Verfahren nach einem der Ansprüche 10 bis 13, **dadurch gekennzeichnet, dass** die nanopartikulären Teilchen eine Größe von 0,01 bis 1,0 µm aufweisen.

15. Verwendung der Trockenpulver, definiert gemäß einem der Ansprüche 1 bis 9 zur Herstellung von Nahrungsergänzungsmitteln sowie als Zusatz zu Lebensmitteln, Tierfuttermitteln, pharmazeutischen und kosmetischen Zubereitungen.

16. Verwendung nach Anspruch 15 zur Herstellung von Weichgelatinekapseln.

17. Nahrungsergänzungsmittel, Lebensmittel, Tierfuttermittel sowie pharmazeutische und kosmetische Zubereitungen, enthaltend Carotinoid-haltige Trockenpulver, definiert gemäß einem der Ansprüche 1 bis 9.

## Claims

1. A dry powder of at least two carotenoids in the form of a multicore structure in which at least two cores of a multicore structure have a different chemical composition.

2. A dry powder as claimed in claim 1, wherein the multicore structure is a particle species having a mean particle size of from 5 to 3000 µm in which the cores are embedded in a matrix.

3. A dry powder as claimed in one of claims 1 or 2 in which the cores have a mean particle size of from 0.01 to 1.0 µm.

4. A dry powder as claimed in one of claims 1 to 3, wherein at least two cores comprise one or more different carotenoids.

5. A dry powder as claimed in one of claims 1 to 4, wherein at least two cores comprise only one representative of the carotenoid class of substances.

6. A dry powder as claimed in one of claims 1 to 5, wherein the carotenoids are a selection from the group of the carotenes and xanthophylls.

7. A dry powder as claimed in one of claims 1 to 6, comprising β-carotene, lycopene and lutein.

8. A dry powder as claimed in claim 7, comprising 1 part by weight of β-carotene, from 0.02 to 20 parts by weight of lycopene and from 0.02 to 20 parts by weight of lutein.

9. A dry powder as claimed in one of claims 1 to 8, having a carotenoid content of from 0.1 to 50% by weight, based on the total amount of the dry powder.

10. A process for producing dry powders defined according to claim 1 by drying an aqueous suspension comprising at least two carotenoids in the form of nanoparticulate particles, which comprises at least two of the nanoparticulate particles having a different chemical composition.

11. A process as claimed in claim 10, wherein at least two of the nanoparticulate particles comprise one or more different carotenoids.

12. A process as claimed in one of claims 10 or 11, wherein at least two of the nanoparticulate particles comprise only one representative of the carotenoid class of substances.

13. A process as claimed in one of claims 10 to 12, wherein the carotenoids are present in the form of protective-colloid-stabilized nanoparticulate particles.

14. A process as claimed in one of claims 10 to 13, wherein the nanoparticulate particles have a size of from 0.01 to 1.0 µm.

15. The use of the dry powder defined according to one of claims 1 to 9 for producing food supplements and as additive to foods, animal feeds, pharmaceutical and cosmetic preparations.

16. The use as claimed in claim 15 for producing soft gelatin capsules.

17. A food supplement, food, animal feed and pharmaceutical and cosmetic preparation comprising carotenoid-containing dry powder defined according to one of claims 1 to 9.

## Revendications

1. Poudre sèche d'au moins deux caroténoïdes sous forme d'une structure à noyaux multiples, **caractérisée en ce qu'**au moins deux noyaux d'une structure à noyaux multiples présentent une composition chimique différente.

2. Poudre sèche suivant la revendication 1, **caractérisée en ce que** la structure à noyaux multiples représente une espèce de particules ayant une taille moyenne de 5 à 3000 µm, dans laquelle les noyaux sont noyés dans une matrice.

3. Poudre sèche suivant l'une des revendications 1 et 2, dans laquelle les noyaux présentent une taille moyenne de 0,01 à 1,0 µm.

4. Poudre sèche suivant l'une des revendications 1 à 3, **caractérisée en ce qu'**au moins deux noyaux contiennent un ou plusieurs caroténoïdes différents.

5. Poudre sèche suivant l'une des revendications 1 à 4, **caractérisée en ce qu'**au moins deux noyaux ne contiennent qu'un représentant de la classe des caroténoïdes.

6. Poudre sèche suivant l'une des revendications 1 à 5, **caractérisée en ce que** les caroténoïdes représentent une sélection parmi le groupe des carotènes et des xanthophylles.

7. Poudre sèche suivant l'une des revendications 1 à 6, contenant du β-carotène, de la lycopine et de la lutéine.

8. Poudre sèche suivant la revendication 7, contenant une partie en poids de β-carotène, 0,02 à 20 parties en poids de lycopine et 0,02 à 20 parties en poids de lutéine.

9. Poudre sèche suivant l'une des revendications 1 à 8, comportant une teneur en caroténoïdes de 0,1 à 50% en poids, par rapport à la quantité globale de la poudre sèche.

10. Procédé de préparation de poudres sèches, définies conformément à la revendication 1, par séchage d'une dispersion aqueuse contenant au moins deux caroténoïdes sous forme de nanoparticules, **caractérisé en ce qu'**au moins deux des nanoparticules présentent une composition chimique différente.

11. Procédé suivant la revendication 10, **caractérisé en ce qu'**au moins deux des nanoparticules contiennent un ou plusieurs caroténoïdes différents.

12. Procédé suivant l'une des revendications 10 et 11, **caractérisé en ce qu'**au moins deux des nanoparticules ne contiennent qu'un représentant de la classe des caroténoïdes.

13. Procédé suivant l'une des revendications 10 à 12, **caractérisé en ce que** les caroténoïdes se présentent sous la forme de nanoparticules stabilisées par un colloïde de protection.

14. Procédé suivant l'une des revendications 10 à 13, **caractérisé en ce que** les nanoparticules présentent une taille de 0,01 à 1,0 µm.

15. Utilisation de la poudre sèche définie conformément à une des revendications 1 à 9, pour la fabrication de compléments alimentaires ainsi que comme additif pour des aliments, des aliments pour animaux, des préparations pharmaceutiques et cosmétiques.

16. Utilisation suivant la revendication 15, pour la fabrication de capsules de gélatine molle.

17. Compléments alimentaires, aliments, aliments pour animaux ainsi que préparations pharmaceutiques et cosmétiques, contenant de la poudre sèche à teneur en caroténoïde, telle que définie suivant l'une des revendications 1 à 9.
